# EUROPEAN PATENT APPLICATION

(11) **EP 4 122 456 A1**
(43) Date of publication of application: **25.01.2023**
(21) Application number: 22180872.8
(22) Date of filing: 24.06.2022
(51) Int. Cl.: A61K 31/045, A61K 36/886, A61K 33/14, A61K 47/36, A61K 9/00, A61P 11/02

(54) **COMPOSITION FOR USE IN THE TREATMENT OF COMMON COLD, PREPARATION METHOD AND USE THEREOF**

(30) Priority: 19.07.2021 US 202117379160
(71) Applicant: Weber, Chuckie, Vancouver, BC V6G 1Y8 (CA)
(72) Inventor: Weber, Chuckie, Vancouver, BC V6G 1Y8 (CA)
(74) Representative: Sonzogni, Laura Gabriella

(57) **Abstract**

The invention relates to a medicinal composition for treating a cold, a preparation method and application thereof. The cold medication composition is made from aloe vera soap, an isopropyl alcohol solution, sea salt, distilled water, and natural gum resin. The invention also relates to a preparation method of the composition and the application of the composition for treating the cold.

## Description

### REFERENCE TO PENDING APPLICATIONS

This application does not claim the benefit of any issued U.S. Patent or pending application.

### TECHNICAL FIELD

The present invention is generally directed toward the cold medication, and more specifically, directed toward the composition for a cold medication utilizing a foam-based composition.

### BACKGROUND

Traditionally, cold and allergy medications are administered in tablets or capsules which for most people are easily swallowed with a glass of water or other beverage. Certain patients however, do not find tablet or capsule dosage forms particularly easy to swallow and therefore are not likely to comply with any therapeutic regimen requiring them. Such patients, in particular children, the aged and the mentally ill who gag, choke or simply refuse to swallow pills or capsules, need other alternative ways to deliver the medication without having to resort to intravenous or intra-muscular injections.

Many medications may be and are orally administrable in liquid elixir and syrup forms but again, these are not often desirable as the liquid carrier may present dissolution problems with respect to the active and/or the active itself may be bitter tasting so that any liquid delivery of the medication tastes terrible. This is often worsened by the lingering after taste as the liquid coats the mouth, throat and tongue. Moreover, the bulky bottles and packaging necessary for liquid preparations are an inconvenience in certain circumstances.

Accordingly, there is a need for a dosage form of medication that address the challenges set out above.

### SUMMARY

The present invention is generally directed toward the cold medication, and more specifically, directed toward the composition for a cold medication utilizing a foam-based composition.

In one aspect, a cold medication composition of the present invention is disclosed. The composition includes a combination of aloe vera soap, an isopropyl alcohol solution, sea salt, distilled water, and natural gum resin.

In another aspect, a method for preparing the cold medication composition incudes combining and blending the composition until it obtains a foam consistency. The composition is then applied to the interior of a nostril proximate to the external opening thereof.

Other aspects and features of the present invention will become apparent to those ordinarily skilled in the art upon review of the following description of specific embodiments of the invention in conjunction with the accompanying figures.

### BRIEF DESCRIPTION OF THE DRAWING

In drawings which illustrate embodiments of the invention wherein similar characters of reference denote corresponding parts in each view,

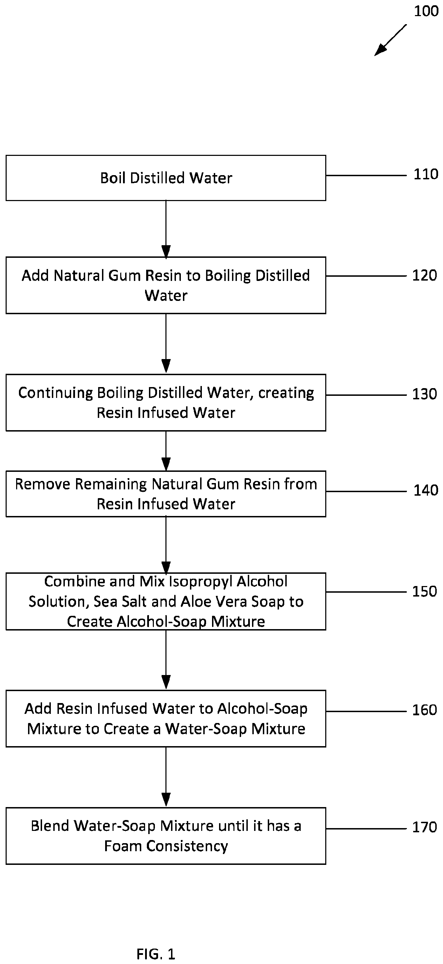

FIG. 1 is a block diagram of an embodiment of a method of preparation of the composition of the present invention.

### DETAILED DESCRIPTION OF THE INVENTION

A cold medication composition for the treatment of cold symptoms, along with methods of preparation and application, are disclosed.

In some embodiments, the cold medication composition disclosed herein is a fast-acting composition capable of reducing or clearing cold symptoms very quickly. Further, in some embodiments, the cold medication composition disclosed herein is capable of reducing or clearing cold symptoms in a two to three minute timeframe from application.

In one embodiment, a cold medication composition is disclosed. In this embodiment, the cold medication composition includes, in combination, aloe vera, soap, isopropyl alcohol solution, sea salt, distilled water, and natural gum resin. The inclusion of the distilled water allows for the composition to be created into a desirable consistency for application into an interior portion of a nasal cavity. The natural gum resin provides additional binding of the composition during the application thereof. The aloe vera soap, isopropyl alcohol solution, and sea salt are utilized to as the active agents in treating the microorganisms causing the cold.

The isopropyl alcohol solution may be present in the composition from between 50% to 95% alcohol and between 5% and 50% purified water. In this embodiment, the isopropyl alcohol solution is in an amount of 90% alcohol and 10% purified water. The use of the 90% isopropyl alcohol solution is intended for use with adults. In an embodiment of the cold medical cold medication composition for use with a child, the isopropyl alcohol solution would include an amount of 60% alcohol and 40% purified water.

The sea salt in the medical cold medication composition may be in an amount between 1/8 teaspoon and 1/2 teaspoon. In this embodiment, the amount of sea salt is 1/4 teaspoon.

The distilled water in the cold medication composition may be between 50 mL and 150 mL. In this embodiment, the amount of distilled water is 100 mL.

The natural gum resin in the cold medication composition may be between 2 g and 10 g. In this embodiment, the amount of natural gum resin is at least 5 g. The natural gum resin may be any form of natural gum resin including chicle or any other natural gum resin such as those found in naturally based chewing gum.

The cold medication composition may create a strong antiseptic smell. In order to help mask that smell, the aloe vera soap may be scented. Further additional scent may be added to the composition to further mask any such antiseptic smell.

As illustrated in figure 1, an embodiment of a method 100 for preparing a cold medication composition is disclosed. In this method and amount of distilled water is heated to a boil, at step 110. The amount of distilled water may be between 50 mL and 100 50 mL, in this embodiment, the amount of distilled water is 100 ml.

At step 120, after the distilled water is brought to a boil, an amount of natural gum resin is added to the boiling distilled water. The amount of natural gum resin may be between two g. and 10 g. In this embodiment, the amount of natural gum resin is at least 5 g.

At step 130, the distilled water/resin combination is allowed to continue to boil for an amount sufficient to create a resin infused water. In this embodiment, the water/resin combination continues to boil for at least two minutes.

At step 140, any remaining natural gum resin is removed from the resin infused water and discarded. The. The resin infused water is then set aside.

At step 150, an isopropyl alcohol solution, sea salt and aloe vera soap are combined and mixed together to create an alcohol soap mixture. The isopropyl alcohol solution may be in an amount of between 50 to 90% alcohol and between five to 50% purified water. In this embodiment, the isopropyl alcohol solution is in the amount of 90% alcohol and 10% purified water. Additionally, the aloe vera soap may or may not be scented. Further, the sea salt may be in an amount of between 1/8 teaspoon and 1/2 teaspoon. In this embodiment, the sea salt is in an amount of 1/4 teaspoon.

At step 160, after the alcohol soap mixture has been created, the resin infused water is then combined with the alcohol soap mixture, creating a water soap mixture. At step 170, the water soap mixture is then blended until it obtains a foam consistency.

An embodiment for the treatment of colds is disclosed. This embodiment utilizes the cold medication composition disclosed above, along with the method of creating that cold medication composition as disclosed above. This embodiment for the treatment of colds includes administering an amount of the cold medication composition to the interior portion of a nostril proximate to the external opening thereof.

While preferred embodiments of the present inventive concept have been shown and disclosed herein, it will be obvious to those persons skilled in the art that such embodiments are presented by way of example only, and not as a limitation to the scope of the inventive concept. Variations, changes, and substitutions may occur or be suggested to those skilled in the art without departing from the intent, scope, and totality of this inventive concept. Such variations, changes, and substitutions may involve other features which are already known per se and which may be used instead of, in combination with, or in addition to features already disclosed herein. Accordingly, it is intended that this inventive concept be inclusive of such variations, changes, and substitutions, and by no means limited by the scope of the claims presented herein.

## Claims

1. A cold medication comprising in combination: aloe vera soap, an isopropyl alcohol solution, sea salt, distilled water, and natural gum resin.

2. The cold medication of claim 1, wherein the aloe vera soap is scented.

3. The cold medication of claim 1, wherein the isopropyl alcohol solution is present in an amount from between fifty percent (50%) to ninety-five percent (95%) alcohol and between five percent (5%) to fifty percent (50%) purified water.

4. The cold medication of claim 1, wherein the isopropyl alcohol solution is present in an amount of ninety percent (90%) alcohol and ten percent (10%) purified water.

5. The cold medication of claim 1, wherein the isopropyl alcohol solution is present in an amount of sixty percent (60%) alcohol and forty percent (40%) purified water.

6. The cold medication of claim 1, wherein the sea salt is present in an amount between one-eighth teaspoon (1/8 tsp) and ½ teaspoon (1/2 tsp).

7. The cold medication of claim 1, wherein the distilled water is present in an amount between fifty milliliters (50ml) and one hundred fifty milliliters (150ml).

8. The cold medication of claim 1, wherein the natural gum resin is present in an amount between two grams (2 grams) and ten grams (10 grams).

9. The cold medication of claim 1, wherein
the isopropyl alcohol solution is present in an amount of ninety percent (90%) alcohol and ten percent (10%) purified water;
the sea salt is present in an amount of one-fourth teaspoon (1/4 tsp);
the distilled water is present in an amount of one hundred milliliters (100ml); and
the natural gum resin is present in an amount of at least five grams (5 grams).

10. A method for preparing a cold medication comprising:
heating an amount of distilled water to a boil;
adding natural gum resin to the boiling distilled water;
continuing to boil the distilled water/natural gum resin combination for an amount of time to create a resin infused water;
removing any remaining natural gum resin from the resin infused water;
combining and mixing together an isopropyl alcohol solution, sea salt and aloe vera soap creating an alcohol/soap mixture;
combining the resin infused water to the alcohol/soap mixture creating a water/soap mixture; and blending the water/soap mixture until the water/soap mixture obtains a foam consistency.

11. A method for the treatment of colds and sinus discomfort comprising:
administering an amount of a cold medication to the interior of a nostril proximate to the external opening thereof,
wherein the cold medication includes aloe vera soap, an isopropyl alcohol solution, sea salt, distilled water, and natural gum resin.
